(19) Europäisches Patentamt
European Patent Office
Office européen des brevets

(11) Publication number: 0 059 903
A1

(12) # EUROPEAN PATENT APPLICATION

(21) Application number: 82101514.6

(22) Date of filing: 27.02.82

(51) Int. Cl.³: C 07 D 271/06
C 07 D 413/12, C 07 D 417/12
C 07 D 413/14, C 07 D 417/14
A 61 K 31/41, A 61 K 31/425
A 61 K 31/535, A 61 K 31/445
A 61 K 31/44, A 61 K 31/54
//(C07D413/12, 271/06, 307/52),
(C07D417/12, 271/06, 277/28),
(C07D413/12, 271/06, 211/26)

(30) Priority: 09.03.81 US 241956

(43) Date of publication of application:
15.09.82 Bulletin 82/37

(84) Designated Contracting States:
AT BE CH DE FR GB IT LI LU NL SE

(71) Applicant: MERCK & CO. INC.
126, East Lincoln Avenue P.O. Box 2000
Rahway, New Jersey 07065(US)

(72) Inventor: Britcher, Susan F.
735 Port Indian Road
Norristown, PA, 19401(US)

(72) Inventor: Lumma, William C.
25 Newman Road M.R. 1
Pennsburg, PA 18073(US)

(74) Representative: Blum, Rudolf Emil Ernst et al,
c/o E. BLUM & CO. Vorderberg 11
CH-8044 Zürich(CH)

(54) **Diaminooxadiazoles, their use as gastric secretion inhibitors, and process for their preparation.**

(57) There are disclosed novel compounds described as diaminooxadiazoles wherein one of the amine functions is connected to a basicly substituted heterocyclic group through a linear or cyclic connecting group. Processes for the preparation of such compounds from the known N-cyano-S-methylisothiourea precursors by treatment with hydroxylamine and for alkylation of amine groups are also disclosed. The compounds are useful for the suppression of gastric acid secretions in mammals and compositions for such uses are also disclosed.

EP 0 059 903 A1

TITLE OF THE INVENTION

Diaminooxadiazoles, their use as Gastric Secretion Inhibitors, and Process for their preparation

BACKGROUND OF THE INVENTION

Compounds containing a heterocycle connected to a cyanoguanidine moiety through a linear or cyclic connecting group are known in the art as H2 histamine receptor inhibitors and active compounds for the suppression of gastric acid secretions. See for example U.S. Patent 3,950,333 to Durant *et al*, U.S. Patent 4,128,658 to Price *et al* and European Patent 3640 to Jones *et al*. In preparing the cyanoguanidine end group, the precursor thereof is an N-cyano-S-methylisothiourea group, which is reacted with the appropriate amine to prepare the described compounds. Applicants have found that the N-cyano-S-methylisothiourea precursor, when reacted with hydroxylamine, produces a compound with an amino-oxadiazole end group.

Belgian Patent 875,846 as abstracted in Derwent Abstract 79110B/44 discloses compounds wherein

a triazolyl heterocyclic ring is incorporated into compounds similar to those disclosed in Durant et al, Price et al and Jones et al in place of the guanidino end group. Such compounds do not suggest, however, the instant oxadiazolyl compounds. The instant oxadiazolyl compounds have been found to possess considerable activity in the suppression of gastric acid secretions.

## SUMMARY OF THE INVENTION

This invention is concerned with diaminooxadiazole compounds which have one of the amino groups connected to an aminoalkyl heterocyclic moiety through a linear or cyclic connecting group. Thus, it is an object of this invention to described such compounds. It is a further object of this invention to describe processes for the preparation of such compounds from the appropriately substituted N-cyano-S-methylisothiourea compound. A still further object is to describe the use of such compounds as gastric secretion inhibitors in mammals. Further objects will become apparent from a reading of the following description.

## DESCRIPTION OF THE INVENTION

The compounds of this invention are best described in the following structural formulae:

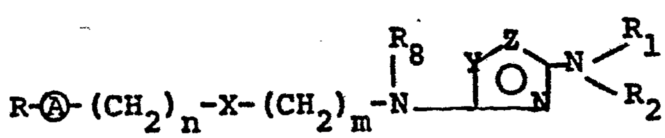

(I)

and

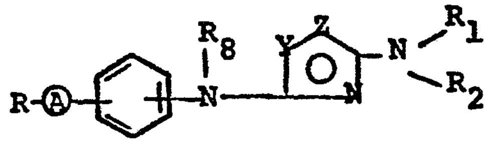

(II)

wherein in both of the above structural formulae:

R is hydrogen, loweralkyl,

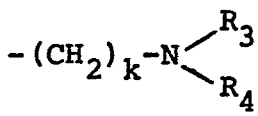 or 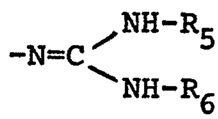

wherein

$R_3$ and $R_4$ are independently hydrogen, loweralkyl, cycloloweralkyl or phenylloweralkyl or $R_3$ and $R_4$ may be joined to form, along with the nitrogen to which they are attached, a 5- or 6-membered heterocycle, which may also contain an oxygen, sulfur or an $N-R_7$ heteroatom, selected from pyrrolidino, piperidino, thiomorpholino, morpholino, or N-loweralkylpiperazino wherein $R_7$ is hydrogen or loweralkyl of from 1 to 3 carbon atoms;

$R_5$ and $R_6$ are independently hydrogen or loweralkyl;

n is 0 or 1;

m is 2 to 4;

k is 1 to 4;

X is oxygen, sulfur or methylene;

Y and Z are oxygen or nitrogen provided they are not the same;

$R_1$ and $R_2$ are independently hydrogen, loweralkyl, loweralkenyl, or loweralkanoyl;

$R_8$ is hydrogen or loweralkyl; and

Ⓐ is phenylene or a 5- or 6-membered heterocycle containing one to three heteroatoms selected from oxygen, sulfur or nitrogen, which may optionally have a benzo ring fused thereon;

provided that when Ⓐ in Formula I above is a 5-membered heterocycle or a benzo fused 5-membered heterocycle, n is 1.

Ⓐ in the foregoing formulae may be furan, thiophene, pyrrole, oxazole, oxadiazole, thiadiazole, thiazole, triazole, pyrazole, imidazole, pyridine, pyrimidine, pyrazine, and the like and the fused benzo derivatives thereof such as benzofuran, benzoxazole, benzimidazole, and the like;

In the instant invention, unless specified otherwise, the term "loweralkyl" is intended to include those alkyl groups containing from 1 to 5 carbon atoms in either a straight or branched configuration. Examples of such alkyl groups are methyl, ethyl, propyl, iso-propyl, butyl, sec-butyl, pentyl and the like.

The term "cycloloweralkyl" is intended to include those cycloalkyl groups of from 3 to 7 carbon atoms. Examples of such cycloalkyl groups are cyclopropyl, cyclobutyl, cyclopentyl, cyclohexyl, and cycloheptyl.

The term "loweralkenyl" is intended to include those alkenyl groups of from 2 to 5 carbon atoms of either a straight or branched configuration and one unsaturation. Examples of such alkenyl groups are ethenyl, propenyl, butenyl, pentenyl, and the like.

The term "loweralkynyl" is intended to include those alkynyl groups of from 2 to 5 carbon atoms of either a straight or branched configuration, and one triple bond. Examples of such alkynyl groups are ethynyl, propargyl, butynyl, pentynyl, and the like.

The preferred compounds of the instant invention will have definitions which vary with the nature of the compounds of Formula I and II.

In Formula I preferred compounds are realized when $R_1$ and $R_2$ are independently hydrogen, loweralkyl, loweralkynyl or substituted loweralkyl wherein the

substituent is phenyl, pyridyl or imidazolyl; and $R_3$ and $R_4$ are independently hydrogen, loweralkyl or $R_3$ and $R_4$ are joined to form a piperidine, morpholine or N-loweralkyl piperazine heterocyclic ring.

Further preferred compounds are realized when $R_1$ and $R_2$ are independently hydrogen or loweralkyl.

Further such preferred compounds are those wherein Ⓐ is m-phenylene or a 6-membered heterocycle as defined above, n is O, X is oxygen, m is 3 and R is:

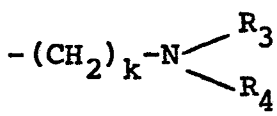

In such cases it is further preferred that k be 1 and $R_3$ and $R_4$ be loweralkyl, preferably methyl, morpholine, piperidine or N-methyl piperazine.

Additional preferred variation of Ⓐ are those wherein Ⓐ is furan, imidazole, thiazole, oxazole, thiophene, triazole, thiadiazole, oxadiazole or benzofuran.

When Ⓐ is heterocyclic it is preferred that n=1, X=sulfur and m=2.

The preferred values of R will depend upon and vary with the definition of Ⓐ.

When Ⓐ is furan or benzofuran, R is preferred to be:

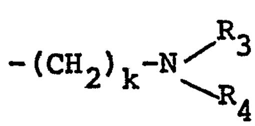

wherein it is further preferred that k=1 and $R_3$ and $R_4$ be hydrogen or loweralkyl, preferably hydrogen or methyl, morpholine, piperidine or N-methyl piperazine.

When Ⓐ is imidazole R is preferably loweralkyl, most preferably methyl.

When Ⓐ is thiazole, R is preferably:

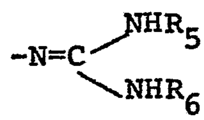

where $R_5$ and $R_6$ are most preferably hydrogen.

In Formula II it is preferred that $R_1$ and $R_2$ are independently hydrogen or loweralkyl.

It is further preferred that the phenyl ring in Formula II be connected to the adjacent groups at its meta positions.

As in Formula I compounds the preferred values of R in Formula II compounds will vary with the nature of Ⓐ. The preferred values of Ⓐ are imidazole, and thiazole.

When Ⓐ is imidazole, it is preferred that R be loweralkyl, most preferably methyl.

When Ⓐ is thiazole, it is preferred that R be:

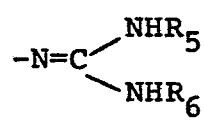

and wherein it is most preferred that $R_5$ and $R_6$ be hydrogen.

The compounds according to the invention readily form physiologically acceptable salts. Such salts include salts with inorganic acids such as hydrochlorides, hydrobromides, sulfates, nitrates and phosphates. Particularly useful salts of organic acids are formed with aliphatic mono- or dicarboxylic acids. Examples of such salts are acetates, maleates fumarates, tartrates, citrates, benzoates, succinates and isethionates. The compounds and their salts may also form hydrates and solvates. In addition, the nitrogen atoms in groups R, Ⓐ $R_1$ and $R_2$ may also form quaternary salts and N-oxides.

As stated above, the compounds represented by formula 1 have been found to have pharmacological activity in the animal body as antagonists to certain actions of histamine which are not blocked by "antihistamines" such as mepyramine. For example, they have been found to inhibit selectively the histamine-stimulated secretion of gastric acid from in the stomach of chronic fistula dogs at doses of from 0.001 to 5 mg per kilogram intravenously, or orally from 0.01 to 10 mg per kilogram. Similarly, the action of these compounds is demonstrated by their antagonism to the effects of histamine on other tissues which are not affected by histamine H1 antagonists. An example of such tissue is the isolated guinea-pig right atrium. The compounds of the invention have also been found to inhibit the secretion of gastric acid stimulated by pentagastrin or by food.

The pharmaceutical carrier employed may be for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the like.

A wide variety of pharmaceutical forms can be employed. Thus, if a solid carrier is used, the preparation can be tableted, placed in a hard gelatin capsule in powder or pellet form, or in the form of a troche or lozenge. The amount of solid carrier will vary widely but preferably will be from about 15 mg to about 0.4 gm. If a liquid carrier is used, the preparation may be in the form of a syrup, emulsion,

soft gelatin capsule, sterile injectable liquid such as an ampoule, or an aqueous of nonaqueous liquid suspension.

The pharmaceutical compositions are prepared by conventional techniques involving procedures such as mixing, granulating and compressing or dissolving the ingredients as appropriate to the desired preparation.

The active ingredient will be present in the composition in an effective amount to inhibit histamine activity. The route of administration may be orally or parenterally.

Préferably, each dosage unit will contain the active ingredient in an amount of from about 1 mg to about 500 mg most preferably from about 20 mg to about 200 mg given in a single dose or multiple divided doses.

For therapeutic use, the pharmacologically active compounds of the present invention will normally be administered as a pharmaceutical composition comprising, as the essential active ingredient, at least one such compound in the basic form or in the form of an addition salt with a pharmaceutically acceptable acid and in association with a pharmaceutical carrier therefor. Such addition salts include those mentioned above.

Other pharmacologically active compounds may in certain cases be included in the composition. It may be appropriate to combine the instant compound or compounds with anticholinergic agents such as propantheline; $H_1$ antihistamines such mepyramine, pyribenzamine, chloropheniramine and the like; or prostanoids.

Advantageously the composition will be made up in a dosage unit form appropriate to the desired mode of administration, for example as a tablet, capsule, or injectable solution.

The compounds of the present invention may be made by reacting a suitably substituted N-cyano-3-methylisothiourea with hydroxylamine optionally followed by alkylation of the derived diaminooxadiazoles as outlined in the following reaction schemes I and II.

REACTION SCHEME I

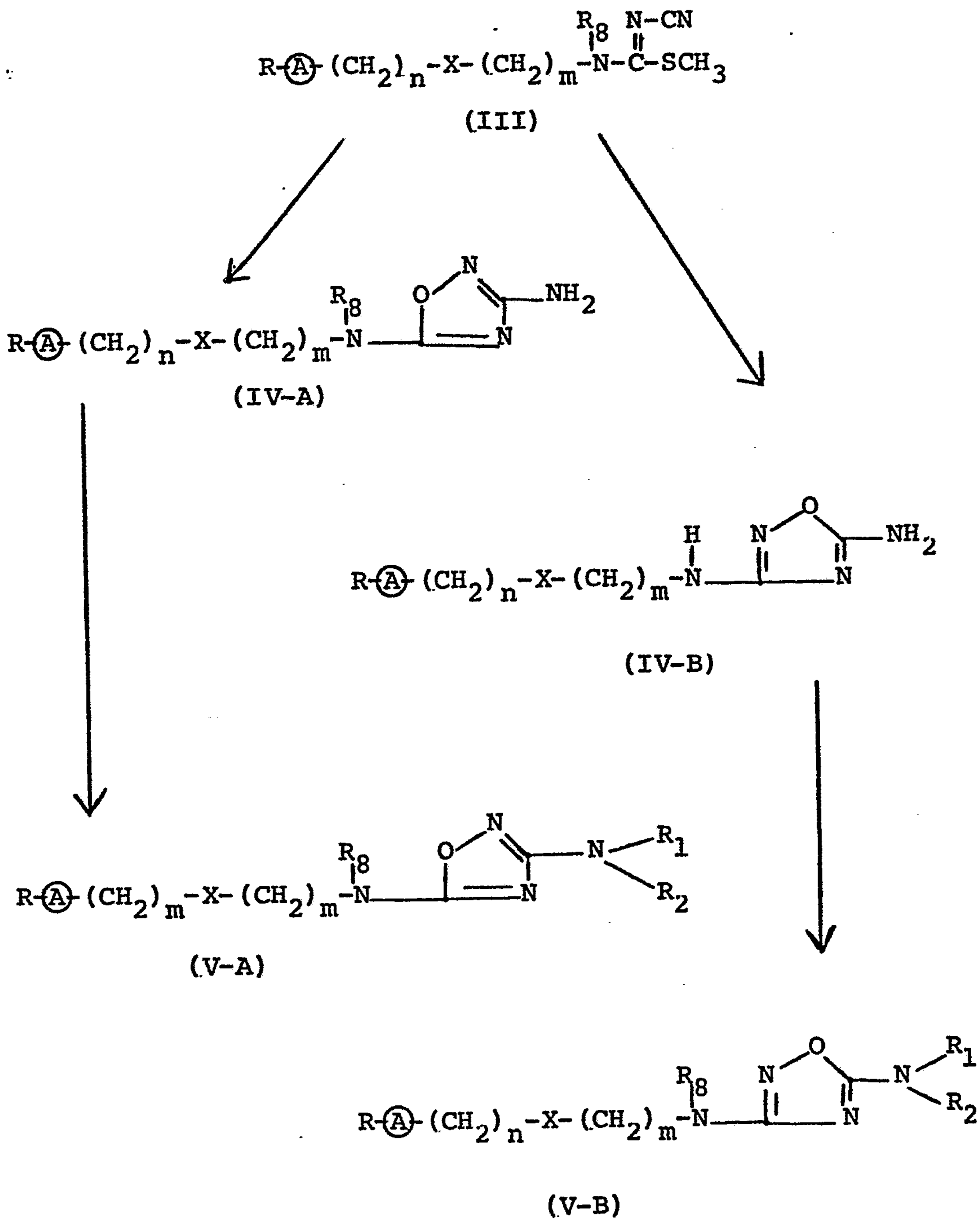

(III)

(IV-A)

(IV-B)

(V-A)

(V-B)

REACTION SCHEME II

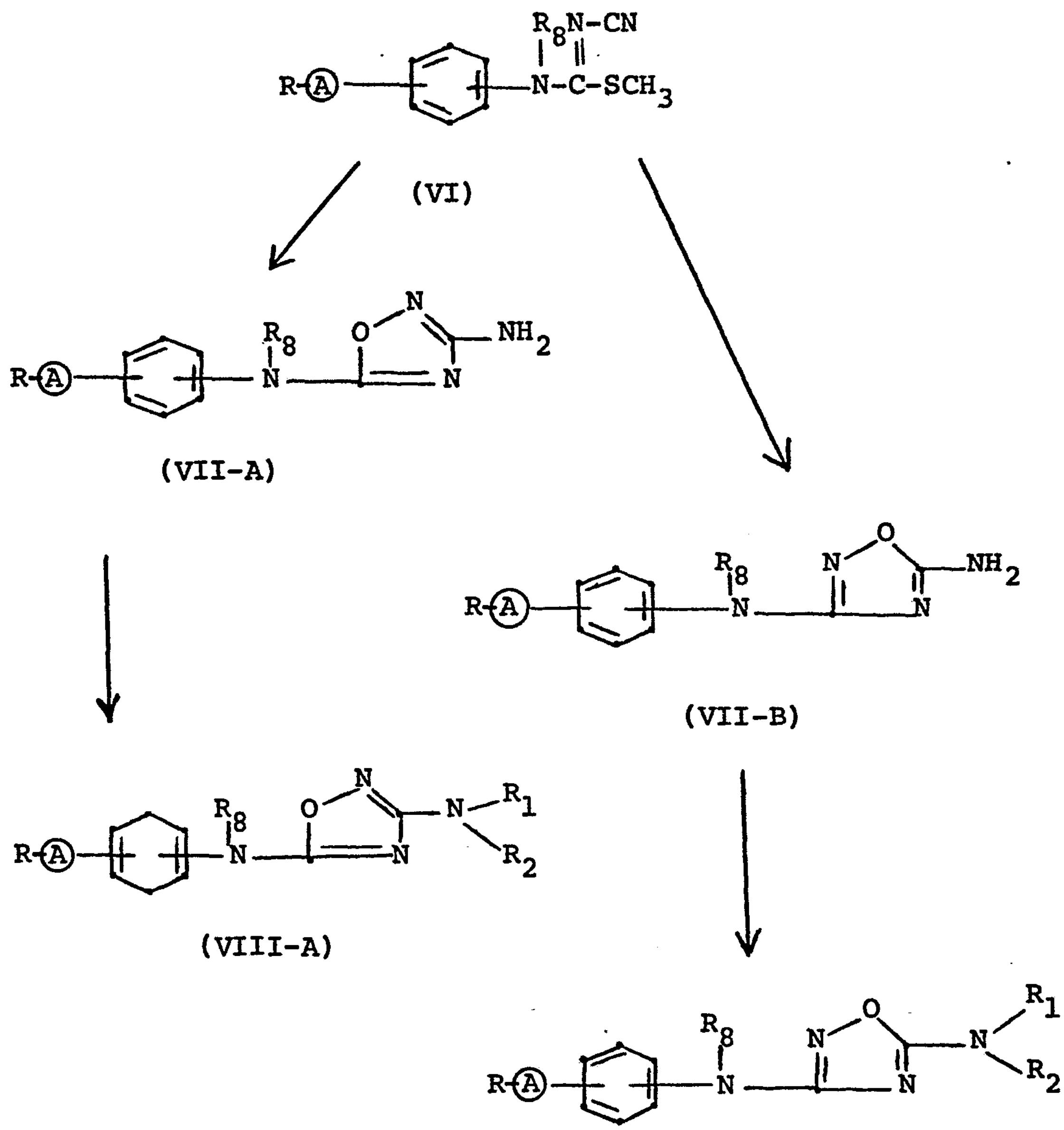

(VI)

(VII-A)

(VII-B)

(VIII-A)

(VIII-B)

wherein R, Ⓐ, X, n, m, $R_1$ and $R_2$ are as previously defined.

The first step of the reaction involves the reaction of the N-cyano-S-methylisothiourea (III) with hydroxylamine. The hydroxylamine is generally added to the reaction mixture as a salt, preferably the hydrochloride salt, and the free base generated in situ. The hydroxylamine free base is liberated using a base added to the reaction mixture. Any base strong enough to remove the salt from the hydroxylamine is suitable and both organic bases such as tertiary amines and inorganic bases have been used. The preferred bases are pyridine, triethylamine, an alkali metal alkoxide such as sodium or potassium methoxides or ethoxide. The base is required to be present in a single molar equivalent, however, excess base has not been found to be detrimental.

Alternatively, the hydroxylamine free base may be pregenerated and added to the reaction mixture without any added base in the form of a solution thereof. The reaction may also be carried out using hydroxylamine salts, however, yields are diminished and variable. It is preferred to carry out the reaction with a hydroxylamine salt and to liberate the free base in situ.

The reaction is carried out in a polar or non-polar solvent and the choice of solvent somewhat determines the nature of the products obtained. If a non-polar solvent is employed, such as tetrahydrofuran, ether, benzene and the like, compound IV-A is the product formed almost exclusively. (That is the product of Formula I wherein Y is O and Z is N). If a polar solvent is used, such as a loweralkanol, preferably methanol or ethanol, a mixture of compounds IV-A and IV-B is obtained. The mixture is readily separated chromatographically using any of the generally

used chromatographic techniques such as thin layer preparative layer, column, high pressure liquid chromatography and the like. The support medium may be alumina, silica gel, ion exchange resins, dextran gels and the like.

With either polar or non-polar solvents, the reaction is carried out from room temperature to the reflux temperature of the reaction medium. It is preferred to carry out the reaction at room temperature. The reaction is slow and reaction times of from 2 days to 2 weeks are not uncommon. The progress of the reaction is conveniently monitored using thin layer chromatographic analysis of an aliquot of the reaction mixture. Using this technique many reactions have been found to reach a point of no further reaction in about 4-5 days. The products are isolated using techniques known to those skilled in the art.

The products of the first step of the reaction have the unsubstituted amine function on the 3-position (IV-A) or 5-position (IV-B) of the oxadiazole moiety. The 3-amino function of IV-A when $R_8$ is other than hydrogen is substituted with the $R_1$ and $R_2$ groups by reaction with a strong base such as an alkali metal alkoxide, preferably sodium or potassium methoxide or ethoxide; or an alkali metal hydride, preferably sodium or potassium hydride. The reaction is carried out in a solvent such as N,N-dimethyl formamide or tetrahydrofuran. The alkali metal salt thus produced is then reacted with an $R_1$ or $R_2$ halide wherein $R_1$ and $R_2$ are as previously defined.

The compounds (IV-A) wherein $R_8$ is loweralkyl are prepared directly from compound III when $R_8$ is loweralkyl or from the compounds (IV-A) wherein $R_8$ is hydrogen using the same procedure as described above for the preparation of those compounds wherein $R_1$ and $R_2$ are other than hydrogen.

Hydrogen atoms on the 5-nitrogen substituent of the diaminooxadiazole are more acidic than those on the 3-nitrogen substituent. Thus, such groups must be blocked with a protecting group before alkylation can be carried out on the 3-nitrogen substituent. Preferred protecting groups are aralkoxy, preferably benzyloxymethyl, prepared from the corresponding halide using conventional techniques. The conversion of IV-A to V-A requires the blocking of the internal 5-nitrogen substituent with similar protecting groups, followed by subsequent alkylation with $R_1$ or $R_2$ halides. The protecting group is removed by acid hydrolysis with aqueous acid.

The N-cyano-S-methylisothiourea starting materials are all known compounds fully described in the prior art discussed above. Thus, processes for their preparation need not be described here.

The following examples are provided in order that this invention might be more fully understood. They are not to be construed as limitative of the invention.

EXAMPLE 1

3-Amino-5-N-[2-[(5-dimethylaminomethyl-2-furanyl)methylthio]ethyl]amino-1,2,4-oxadiazole and 5-amino-3-N-[2-[(5-dimethylaminomethyl-2-furanyl)methylthio]ethyl]-amino-1,2,4-oxadiazole

A solution of 4.90 g (15.7 mmole) of N-cyano-N'-2-[5-(dimethylaminomethyl)2-furanylmethylthio)ethyl-S-methylisothiourea, 3.85 g (55 mmole) of hydroxylamine hydrochloride, and 7.7 ml (5.6 g, 55 mmole) of triethylamine in 15 ml of absolute methanol is stirred gently at ambient temperature for four days or until the disappearance of starting material by thin layer chromatography is observed. The thin layer chromatography system is alumina GF, and elution is with 95% chloroform, 5% methanol.

The reaction solution is concentrated to an oil on a film evaporator and the oil is partitioned between ethyl acetate and dilute aqueous sodium carbonate solution. Following two additional ethyl acetate extractions of the aqueous phase the combined organic layers are dried over sodium sulfate, filtered and evaporated in vacuo to an oil which is a mixture of two thin layer chromatography spots, with Rf valves of 0.62 and 0.47. The mixture is chromatographed on a column of neutral alumina (90 g, activity III), eluting with chloroform. Separation of the two components is accomplished. The major component is an oil, 900 mg with a thin layer chromatography Rf of 0.62. Mass spectral and $^{13}C$, $^{1}H$ NMR data confirm the structure as 3-amino-5-N-[2-[(5-dimethylaminomethyl-2-furanyl)methylthio]ethyl]amino-1,2,4-oxadiazole. This oil, on standing, crystallizes to a white solid, mp 88-89°C; this is identical with the material prepared in tetrahydrofuran (Example 2). Nuclear magnetic resonance spectroscopy of this material reveals the following characteristic peaks. Resonances were taken in $CDCl_3$ and are given in $\delta$ relative to tetramethylsilane: $^{13}C$-nuclear magnetic resonance 170.1 and 168.2 (oxadiazole carbons) $^{1}H$-nuclear magnetic resonance 4.55 (singlet, 2 protons from $-NH_2$) 7.46 (triplet, 1 proton from -NH.

The minor component is also an oil, 500 mg with a thin layer chromatography Rf of 0.47. Mass spectral and $^{13}C$, $^{1}H$ NMR data confirm the structure 5-amino-3-N-[2-[(5-dimethylaminomethyl-2-furanyl)methylthio]ethyl]amino-1,2,4-oxadiazole. Nuclear magnetic resonance spectroscopy of this material reveals the following characteristic peaks. Resonances were taken in $CDCl_3$ and are given in $\delta$ relative to tetramethylsilane: $^{13}C$-nuclear magnetic resonance 170.2 and 168.5 (oxadiazole carbons) $^{1}H$ nuclear magnetic resonance 5.69 (triplet, 1 proton from -NH) 6.52 (singlet, 2 protons from $-NH_2$).

EXAMPLE 2

3-Amino-5-N-[2-[(5-dimethylaminomethyl-2-furanyl)methylthio]ethyl]amino-1,2,4-oxadiazole

A solution of N-cyano-N'-2-[5-(dimethylaminomethyl)-2-furanylmethylthio]ethyl-S-methylisothiourea (9.37 g, 0.030 mole) in 150 ml of dry tetrahydrofuran is treated with 3.00 g (0.045 mole) of hydroxylamine hydrochloride and 6.00 ml (0.045 mole) of triethylamine and stirred for 8 days at ambient temperature. The mixture is concentrated under vacuum and the residue is partitioned between 100 ml of ethyl acetate and 50 ml of saturated sodium bicarbonate solution. 25 Ml of saturated sodium carbonate solution is added to the aqueous layer and it is extracted with two 100 ml portions of ethyl acetate. The organic layers are combined, dried over anhydrous sodium sulfate, filtered, and concentrated under vacuum to give 10.46 g of an oil. The oil is purified by column chromatography using activity III neutral alumina. The product is contained in fractions eluted by chloroform, followed by 1% methanol and 10% methanol in chloroform. Concentration of the fractions under high vacuum affords 5.60 g of crude solid material. Recrystallization from water yields 3.26 g of 3-amino-5-N-[2-[(5-dimethylaminomethyl-2-furanyl)methylthio]ethyl]amino-1,2,4-oxadiazole, mp 88-89°C. The nuclear magnetic resonance spectrum of this compound is identical with the 3-amino isomer prepared in Example 1.

EXAMPLE 3A

3-Amino-5-[N-methyl-N-[2-[(5-dimethylaminomethyl-2-furanyl)methylthio]ethyl]amino-1,2,4-oxadiazole

A mixture of 3-amino-5-N-[2-(5-dimethylamino-methyl)-2-furanylmethylthio]ethylamino-1,2,4-oxadiazole (1.5 g, 5.0 mmole) and 250 mg (5.0 mmole) of a 50% sodium hydride dispersion in 3 ml of N,N-dimethylformamide is rapidly agitated with mechanical stirring and warming under a nitrogen atmosphere. Hydrogen gas evolution and formation of a deep red solution are immediately noted after which the mixture is stirred at ambient temperature for an additional 15 minutes. Methyl iodide (0.32 ml, 5.0 mmole) is introduced via a syringe through a rubber septum and stirring of the resulting tan suspension is continued for 15 minutes. The mixture is diluted with 20 ml of water and the aqueous solution extracted with methylene chloride (3 X 10 ml). The combined organic extracts are extracted with two 10 ml portions of 1N hydrochloric acid and the combined aqueous acidic phases are then washed with methylene chloride before basification with solid sodium carbonate. Methylene chloride extraction of the alkaline aqueous phase gives 1.8 g of an orange oil which is purified by chromatography through a neutral alumina (activity III) column, elution with methylene chloride followed by 2% methanol in methylene chloride. Concentration of the fractions gives 1.6 g of 3-amino-5-[N-methyl-N-[2-[(5-dimethylaminomethyl-2-furanyl)methylthio]ethyl]amino]-1,2,4-oxadiazole.

EXAMPLE 3B

3-(2-Guanidino-4-thiazolyl)-N-methyl benzeneamine

A mixture of 9.3 g (.04 mole) of 3-(2-guanidino-4-thiazolyl)benzenamine, 20 ml of triethylorthoformate, and 2 drops of trifluoroacetic acid is stirred at reflux for 5 hours after which it is concentrated in vacuo. The residue is dissolved in 20 ml of ethanol, the solution cooled to 0° C and then treated with excess sodium borohydride. The mixture is allowed to warm to room temperature, then stirred at reflux for 4 hours. Upon concentration in vacuo the residue is partitioned between water and dichloromethane.

After three extractions of the aqueous phase the combined dichloromethane extracts are dried over sodium sulfate and evaporated to give 3-(2-guanidino-4-thiazolyl) -N-methyl benzenamine.

EXAMPLE 3C

N-Cyano-N'-methyl-N'-3(2-guanidino-4-thiazolyl)phenyl-S-methyl isothiourea

A solution of 5.2 g (.021 mole) of 3-(2-guanidino-4-thiazolyl)-N-methylbenzeneamine and 3.1 g (.021 mole) of dimethylcyanodithioimidocarbonate in 100 ml of isopropanol is gently stirred under nitrogen for 18 hours. After evaporation of the solvent the residue is chromatographed using activity II neutral alumina and the product-containing fractions, eluted with dichloromethane, are combined. Upon removal of the solvent there is obtained 6.1 g of N-cyano-N'-methyl-N'-3(guanidino-4-thiazolyl)phenyl-S-methylisothiourea.

EXAMPLE 3D

3-Methylamino-5N-[3-(2-guanidino-4-thiazolyl)phenyl]amino-1,2,4-oxadiazole

Sodium hydride (0.24g, 50% oil dispersion)

(.0050 mole) is suspended in 10 ml of dry dimethylformamide. The mixture is stirred under nitrogen, and then treated with 1.57 g (.0050 mole) of 3-amino-5N-[3-(2-guanidino-4-thiazolyl)phenyl]amino-1,2,4-oxadiazole. After two hours, .080 ml (.0055 mole) of chloro methylpivalate is added and the resultant mixture stirred at ambient temperature for 18 hours. The mixture is cooled to 10°C, treated with additional sodium hydride (0.24 g, 50% oil dispersion) (.0050 mole) and allowed to stir at 25°C for 3 hours. Iodomethane (0.32 ml, 0.0050 mole) is added and vigorous stirring is continued for one hour after which time 6 ml of a 4M sodium hydroxide solution is added dropwise. The mixture is stirred at 25°C for 5 minutes and then poured into excess aqueous sodium bicarbonate. Extractions with dichloromethane provide 3-methylamino-5N-[3-(2-guanidino-4-thiazolyl)phenyl] amino-1,2,4-oxadiazole as an oil.

## EXAMPLE 4

**3-Amino-5-N-[3-(2-guanidino-4-thiazolyl)phenyl]amino-1,2,4-oxadiazole**

To a suspension of 3.31 g (0.0100 mole) of N-cyano N'-3-(2-guanidino-4-thiazolyl)phenyl-S-methylisothiourea in 50 ml dry tetrahydrofuran is added 1.04 g (0.0150 mole) of hydroxylamine hydrochloride and 1.52 g (0.0150 mole) of triethylamine. The mixture is then heated at reflux for 21 hours, at which time no further change is noted by thin layer chromatography. The solution is concentrated to dryness *in vacuo*, and the residue is washed with 50 ml of saturated sodium bicarbonate solution and 50 ml of ethyl acetate and dried to give 2.41 g (77%) of crude product.

The crude product is placed in 5 ml of water and 7.7 ml of 1N hydrochloric acid is added. The solution is filtered and concentrated *in vacuo* until a

precipitate begins to form. Additional water is then added until the solid dissolves. Addition of acetonitrile to the aqueous solution causes precipitation of the product. 1.12 G of the hydrochloride salt is obtained.

The hydrochloride salt is dissolved in 5 ml of water and basified with concentrated ammonia to give 0.90 g of material containing product and origin material according to thin layer chromatography. The material is eluted through a small pad of E. Merck silica gel 60 by a solution of 20% methanol 80% chloroform. The material obtained is then dissolved in 1 ml of dimethylsulfoxide and precipitated by the addition of water to give 0.41 g (13%) of product, m.p. 240-242°, thin layer chromatography (20% methanol/ 80% chloroform, silica gel) Rf 0.2.

Following the foregoing procedures, the listed N-cyano-S-methylisothiourea intermediates are prepared from the amine reactant and dimethylcyanodithioimidocarbonate. Reaction of such intermediates with hydroxylamine affords the oxadiazoles.

Typical compounds encompassed by this invention include:

| Oxadiazole | Oxadiazole Precursor | Amine Reactant |
|---|---|---|
| 3-Methylamino-5N-[2-[(5-dimethylaminomethyl-2-furanyl)methylthio]ethyl]-amino-1,2,4-oxadiazole | 3-Amino-5N-[2-[(5-dimethylaminomethyl-2-furanyl)methylthio]ethyl]-amino-1,2,4-oxadiazole x | 2-[(5-Dimethylamino-methyl-2-furanyl)methyl-thio]ethylamine |
| 5-Amino-3[N-methyl-N-[2-[(5-dimethylaminomethyl-2-furanyl)methylthio]ethyl] amino-1,2,4-oxadiazole | N-Cyano-N'-methyl-N'-[2-[(5-dimethylaminomethyl-2-furanyl)methylthio]-ethyl]-S-methylisothiourea | N-Methyl-N-[2-[(5-Dimethyl-2-furanyl)methylthio] methylethyl]amine |
| 3-Amino-5N-[2-[(5-guanidino-4-thiazolyl)-methylthio]ethyl]amino-1,2,4-oxadiazole | N-Cyano-N'-2-[(2-guanidino-4-thiazolyl)methylthio]ethyl-S-methylisothiourea | 2-[(2-Guanidino-4-thiazolyl)-methylthio]ethylamine |
| 3-Amino-5N-[2-[(6-(4-morpholinomethyl)-2-benzofuranyl)methylthio]-ethyl]amino-1,2,4-oxadiazole | N-Cyano-N'-2-[(6-(4-morpholinomethyl)-2-benzo-furanyl)methylthio]ethyl-S-methylisothiourea | 2-[(6-(4-Morpholinomethyl)-2-benzofuranyl)methylthio]-ethylamine |
| 3-Amino-5N-[3-[3-(4-morpholinomethyl)phenoxy]-propyl]amino-1,2,4-oxadiazole | N-Cyano-N'-3-[3-(4-morpholinomethyl)phenoxy]-propyl-S-methylisothiourea | 3-[3-(4-Morpholinomethyl)-phenoxy]propaneamine |

x Subjected to successive alkylative blocking of 5N-, alkylation of 3N-, then deblocking of 5N.

| Oxadiazole | Oxadiazole Precursor | Amine Reactant |
|---|---|---|
| 5-Amino-3N-[3-[3-(1-piperidinomethyl)phenoxy]-propyl]amino-1,2,4-oxadiazole | N-Cyano-N'-3-[3-(1-piperidino-methyl)phenoxy]propyl-S-methyl isothiourea | 3-[3-(1-Piperidinomethyl)-phenoxy]propaneamine |
| 3-Amino-5N-[2-[(5-methyl-4-imidazolyl)methylthio]-ethyl]amino-1,2,4-oxadiazole | N-Cyano-N'-2-[5-methyl-4-imidazolyl)methylthio]-ethyl-S-methylisothiourea | 2-[(5-Methyl-4-imidazolyl)-methylthio]ethylamine |
| 5-Amino-3N-[2-[(5-methyl 4-imidazolyl)methylthio]-ethyl]-amino-1,2,4-oxadiazole | " | " |
| 3-Amino-5N-[2-[(6-dimethylaminomethyl-2-pyridyl)methylthio]-ethyl]amino-1,2,4-oxadiazole | N-Cyano-N'-2-[(6-dimethyl-aminomethyl-2-pyridyl)methyl-thio]ethyl-S-methylisothio-urea | 2-[(6-Dimethylaminomethyl-2-pyridyl)methylthio]-ethyl-amine |
| 5-Amino-3-N-[[3-2-guanidino-4-thiazolyl)phenyl]N-methyl] amino-1,2,4-oxadiazole | N-Cyano-N'-methyl-N'-3-(2-guanidino-4-thiazolyl)phenyl-S-methylisothiourea | 3-(2-Guanidino-4-thiazolyl) N-methylbenzeneamine |

EXAMPLE 14

N-2[5-Dimethylaminomethyl-2-furanyl)methylthio]ethyl-cyanamide

2-[5-Dimethylaminomethyl-2-furanyl)methylthio] ethylamine (21.4 g, 0.10 mole) and hexamethyldisilazane (30 g) are combined and refluxed vigorously under nitrogen for 18 hours. The resulting mixture is distilled *in vacuo* to give N-trimethylsilyl-2-[(5-dimethylaminomethyl-2-furanyl)methylthio]ethyl amine, bp 95-96° (0.1 mm) as a clear colorless liquid.

This compound (14.4 g, 0.050 mol) is dissolved in 100 ml of methylene chloride at -20°C under nitrogen and the stirred solution treated dropwise with a solution of cyanogen bromide (5.3 g, 0.05 mole) in 50 ml of methylene chloride. The resulting solution is stirred for one hour at -20°C then concentrated *in vacuo* at 10-20°C to give crude N-2[5-Dimethylaminomethyl-2-furanyl)methylthio]ethyl-cyanamide.

EXAMPLE 15

2-Hydroxy-1-[2-[(5-dimethylaminomethyl-2-furanyl]methylthio]ethyl guanidine

The cyanamide from Example 14 (9.56 g, 40 mmol) is added to a stirred suspension of hydroxylamine hydrochloride (4.0 g, 58 mmol) and sodium acetate trihydrate (8.2 g, 60 mmol) in 400 ml of N,N-dimethylformamide. The mixture is stirred overnight at 60°C under nitrogen. The mixture is diluted with water and extracted with ethyl acetate. The extracts are combined and washed with saturated aqueous sodium chloride solution and dried over anhydrous potassium carbonate. Concerntration of the filtered ethyl acetate solution in vacuo gives crude 2-Hydroxy-1[2-[(5-dimethylaminomethyl-2-furanyl)methylthio]ethyl guanidine.

EXAMPLE 16

3-[2-[(5-Dimethylaminomethyl-2-furanyl)methylthio]ethyl] amino-5-trichloromethyl-1,2,4-oxadiazole

The guanidine from Example 15 (5 g, 18 mmol) is dissolved in 75 ml of tetrahydrofuran at 0°C and the solution treated dropwise with trichloracetic anhydride (3.7 g, 20 mmol). The mixture is warmed to room temperature overnight under an atomosphere of dry nitrogen. Most of the solvent is removed in vacuo and the residue is partitioned between aqueous sodium carbonate and methylene chloride. The methylene chloride extract is dried over sodium sulfate, filtered and concentrated in vacuo to an oil which is chromatographed on silica gel to give pure 3-[2-[(5-Dimethylaminomethyl-2-furanyl)methylthio]ethyl] amino-5-trichloromethyl-1,2,4-oxadiazole.

EXAMPLE 17

3-[2-[(5-Dimethylaminomethyl-2-furanyl)methylthio] ethyl]amino-5-methylamino-1,2,4-oxadiazole

The oxadiazole from Example 16 (3 g, 7.5 mmol) is dissolved in 25 ml of ethanol saturated with methylamine and the solution heated in a sealed tube at 50°C for 16 hours. The mixture is concentrated to provide 3-[2-[(5-Dimethylaminomethyl-2-furanyl)methylthio] ethyl]amino-5-methylamino-1,2,4-oxadiazole as an oil.

WHAT IS CLAIMED IS:

1. A compound having the formula:

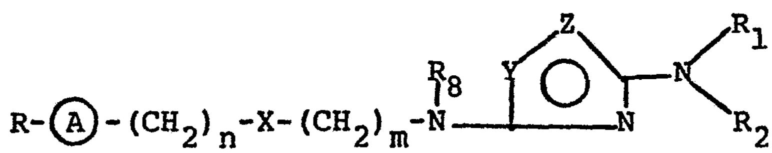

wherein R is hydrogen, loweralkyl:

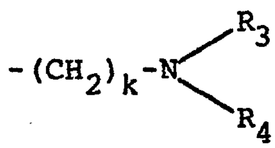

or

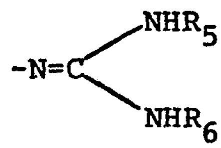

wherein $R_3$ and $R_4$ are independently hydrogen, loweralkyl, cycloloweralkyl, or phenylloweralkyl, or $R_3$ and $R_4$ may be joined to form, along with the nitrogen to which they are attached, a 5- or 6-membered heterocycle, which may also contain an oxygen, sulfur or N-$R_7$ heteroatom, wherein $R_7$ is hydrogen, loweralkyl of from 1 to 3 carbon atoms or benzyl;

$R_5$ and $R_6$ are independently hydrogen or loweralkyl;

n is 0 or 1;

m is 2 to 4;

k is 1 to 4

X is oxygen, sulfur or methylene;

Y and Z are oxygen or nitrogen provided they are not the same;

$R_1$ and $R_2$ are independently hydrogen, loweralkyl, loweralkenyl, or loweralkanoyl;

$R_8$ is hydrogen or loweralkyl; and

(A) is phenylene or a 5- or 6-membered heterocycle containing one to three heteroatoms selected from oxygen, sulfur or nitrogen, which may optionally have a benzo ring fused thereon;

provided that when (A) is a 5-membered heterocycle or benzo fused 5-membered heterocycle, n is 1.

2. The compounds of Claim 1 wherein the heterocycle formed when $R_1$ and $R_2$ are joined, or when $R_3$ and $R_4$ are joined is a pyrrolidine, piperidine, morpholine, thiomorpholine or piperazine; or, $R_1$ and $R_2$ are independently hydrogen, loweralkyl, loweralkynyl or substituted loweralkyl, wherein the substituent is phenyl, pyridyl, or imidazolyl; or $R_3$ and $R_4$ are independently hydrogen, loweralkyl, or $R_3$ and $R_4$ are joined to form a piperidine, morpholine, or N-loweralkyl piperazine heterocyclic ring; and (A) is phenylene, furan, thiophene, pyrrole, oxazole, oxadiazole, thiazole, thiadiazole, triazole, pyrazole, imidazole, pyridine, pyrimidine, pyrazine or benzofuran.

3. A compound of Claim 1 which is a member of the group:

3-amino-5-N-[2-[(5-dimethylaminomethyl-2-furanyl)-methylthio]-ethyl]amino-1,2,4-oxadiazole;

5-amino-3-N-[2-[(5-dimethylaminomethyl-2-furanyl)-methylthio]-ethyl]amino-1,2,4-oxadiazole; and,

3-amino-5-N-[3-(2-guanidino-4-thiazolyl)methylthio ethyl]amino-1,2,4-oxadiazole.

4. A compound having the formula:

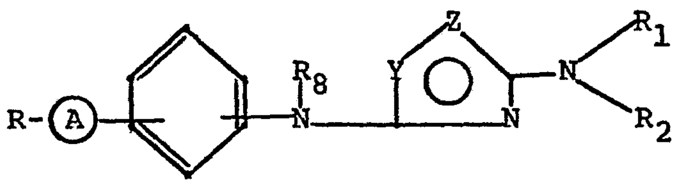

wherein R is hydrogen, loweralkyl,

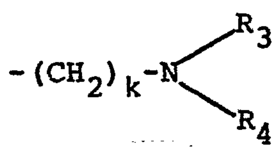

or

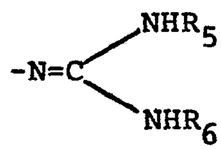

wherein $R_3$ and $R_4$ are independently hydrogen, loweralkyl, cycloloweralkyl, or phenylloweralkyl, or $R_3$ and $R_4$ may be joined to form, along with the nitrogen to which they are attached, a 5- or 6-membered heterocycle, which may also contain an oxygen, sulfur or N-$R_7$ heteroatom, wherein $R_7$ is hydrogen, loweralkyl of from 1 to 3 carbon atoms or benzyl;

$R_5$ and $R_6$ are independently hydrogen or loweralkyl;

k is 1 to 4;

Y and Z are oxygen or nitrogen provided they are not the same;

$R_1$ and $R_2$ are independently hydrogen, loweralkyl, loweralkenyl, or loweralkanoyl;

$R_8$ is hydrogen or loweralkyl; and

Ⓐ is phenylene or a 5- or 6-membered heterocycle containing one to three heteroatoms selected from oxygen, sulfur or nitrogen, which may optionally have a benzo ring fused thereon.

5. The compound of Claim 4 wherein the heterocycle formed when $R_3$ and $R_4$ are joined, is a pyrrolidine, piperidine, morpholine, thiomorpholine, or piperazine; or, $R_1$ and $R_2$ are independently hydrogen, or loweralkyl, and $R_3$ and $R_4$ are independently hydrogen, loweralkyl or $R_3$ and $R_4$ are joined to form a piperidine, morpholine or N-loweralkyl-piperazine heterocyclic ring; and, (A) is oxazole, oxadiazole, thiazole, thiadiazole, triazole, or imidazole.

6. A compound of Claim 4 which is 3-amino-5-N-[3-(2-guanidino-4-thiazolyl)phenyl]amino-1,2,4-oxadiazole.

7. A process for the preparation of a compound of Claim 1 which comprises reacting a compound having the formula:

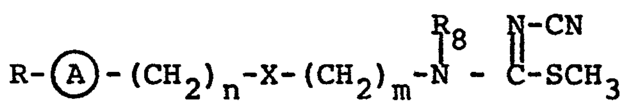

wherein R, $R_8$, (A), X, n and m are as previously defined; with hydroxylamine to prepare the compound:

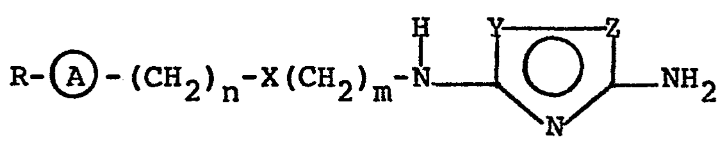

wherein Y and Z are as defined in Claim 1; which is optionally reacted with $R_1$, $R_2$ halides or an $R_8$ halide in the presence of a strong base to prepare the desired product, wherein $R_1$, $R_2$ and $R_8$ are as defined in Claim 1 except that they are not hydrogen.

8. A process for the preparation of a compound of Claim 4 which comprises reacting a compound having the formula:

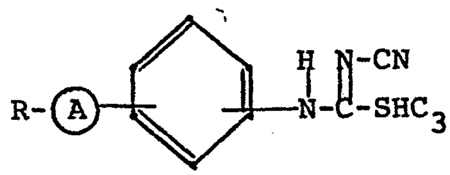

wherein R and (A) are as defined in Claim 1, with hydroxylamine to prepare the compound:

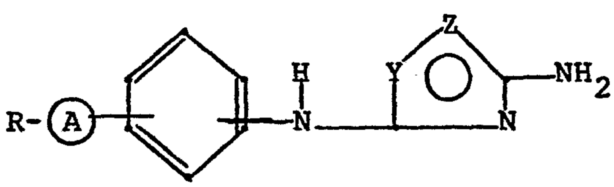

wherein Y and Z are as defined in Claim 1; which is optionally reacted with $R_1$, $R_2$ halides or an $R_8$ halide, in the presence of a strong base, to prepare the desired product, wherein $R_1$, $R_2$ and $R_8$ are as defined in Claim 1 except that they are not hydrogen.

9. A method of suppressing gastric acid secretions in an animal with excess gastric acid secretions which comprises administering to said animal an effective amount of a compound of Claim 1 or Claim 4.

10. A composition useful for suppressing gastric acid secretions in an animal with excess gastric acid secretions which comprises an inert carrier and an effective amount of a compound of Claim 1 or Claim 4.

European Patent Office

# PARTIAL EUROPEAN SEARCH REPORT

which under Rule 45 of the European Patent Convention shall be considered, for the purposes of subsequent proceedings, as the European search report

0059903

Application number

EP 82101514.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.³) |
|---|---|---|---|
| A | CHEMICAL ABSTRACTS, vol. 93, no. 23, Dezember 8, 1980, Columbus, Ohio, USA<br>JEFFERSON W. TILLEY, HENRI RAMUZ "The synthesis of 3,5-diamino-1,2,4-oxadiazoles. Part I" page 524, column 1, abstract no. 220 664 e<br>& Helv. Chim. Acta 1980, 63(4), 832-40<br>-- | 1 | C 07 D 271/06<br>C 07 D 413/12<br>C 07 D 417/12<br>C 07 D 413/14<br>C 07 D 417/14<br>A 61 K 31/41<br>A 61 K 31/425<br>A 61 K 31/535<br>A 61 K 31/445<br>A 61 K 31/44<br>A 61 K 31/54<br>A 61 K 31/495//<br>(C 07 D 413/12<br>C 07 D 271/06 |
| A | US - A - 3 574 222 (ELOY)<br>* Example 29 *<br>-- | 1 | TECHNICAL FIELDS SEARCHED (Int. Cl.³) |
| A | DE - B2 - 1 445 409 (ARON)<br>* Claim *<br>-- | 1 | C 07 D 271/00<br>C 07 D 417/00<br>C 07 D 413/00 |
| A | US - A - 4 089 966 (COHEN)<br>* Example 11 *<br>---- | 1 | |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8,10

Claims searched incompletely: –

Claims not searched: 9

Reason for the limitation of the search: Method for treatment of the human or animal body by therapy (article 52(4) EPC)

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| Place of search | Date of completion of the search | Examiner |
|---|---|---|
| VIENNA | 06-05-1982 | HAMMER |

0059903

European Patent Office

# PARTIAL EUROPEAN SEARCH REPORT

Application number

EP 82101514.6

| DOCUMENTS CONSIDERED TO BE RELEVANT | | | CLASSIFICATION OF THE APPLICATION (Int Cl.³) |
|---|---|---|---|
| Category | Citation of document with indication, where appropriate, of relevant passages | Relevant to claim | |
| | | | C 07 D 307/52)<br>(C 07 D 417/12<br>C 07 D 271/06<br>C 07 D 277/28)<br>(C 07 D 413/12<br>C 07 D 271/06<br>C 07 D 211/26) |
| | | | TECHNICAL FIELDS SEARCHED (Int. Cl.³) |
| | | | |

EPO Form 1505.3 06.78